# EUROPEAN PATENT SPECIFICATION

(11) **EP 2 535 715 B1**
(45) Date of publication and mention of the grant of the patent: **13.09.2017**
(21) Application number: 11742179.2
(22) Date of filing: 07.02.2011
(51) Int. Cl.: G01N 33/574, G01N 33/53, G01N 33/564

(54) **METHOD FOR MEASURING IMMUNITY OF COMPLEX OF Ku86 AND AUTOANTIBODY THEREOF, KIT USED THEREFOR, AND METHOD FOR DETERMINING CANCER USING SAME**
VERFAHREN ZUR MESSUNG DER IMMUNITÄT EINES KU86-KOMPLEXES UND EINES AUTOANTIKÖRPERS DARAUS, DAFÜR VERWENDETES KIT UND VERFAHREN ZUR KREBSDIAGNOSE DAMIT
PROCÉDÉ DE MESURE DE L'IMMUNITÉ DU COMPLEXE ASSOCIANT Ku86 ET UN DE SES AUTO-ANTICORPS, NÉCESSAIRE UTILISÉ À CET EFFET ET MÉTHODE DE DIAGNOSTIC DU CANCER L'UTILISANT

(30) Priority: 12.02.2010 JP 2010028387
(43) Date of publication of application: 19.12.2012
(73) Proprietor: Nitto Boseki Co., Ltd., Gonome Fukushima-shi Fukushima 960-8161 (JP)
(72) Inventor: KOJIMA Ryo, Koriyama-shi Fukushima 963-8061 (JP); NODA Kenta, Koriyama-shi Fukushima 963-8061 (JP); SEIMIYA Masanori, Chiba-shi Chiba 260-8670 (JP); SOGAWA Kazuyuki, Chiba-shi Chiba 260-8670 (JP); NOMURA Fumio, Chiba-shi Chiba 260-8670 (JP)
(74) Representative: Hoffmann Eitle
(86) International application number: PCT/JP2011/052481
(87) International publication number: WO 2011/099435

(56) References cited:
- WO-A1-2011/090017
- WO-A2-2004/048523
- WO-A2-2007/115829
- F. NOMURA ET AL: "Serum anti-Ku86 is a potential biomarker for early detection of hepatitis C virus-related hepatocellular carcinoma", BIOCHEMICAL AND BIOPHYSICAL RESEARCH COMMUNICATIONS, vol. 421, no. 4, 25 April 2012 (2012-04-25), pages 837-843, XP028489195, Elsevier Inc New York NY USA ISSN: 0006-291X, DOI: 10.1016/J.BBRC.2012.04.099 [retrieved on 2012-04-25]
- YANEVA ET AL.: 'Antibodies against Ku protein in sera from patients with autoimmune diseases' CLIN.EXP.IMMUNOL. vol. 76, 1989, pages 366 - 372, XP008161284
- GULLO ET AL.: 'The biology of Ku and its potential oncogenic role in cancer' BIOCHIMICA ET BIOPHYSICA ACTA vol. 1765, 2006, pages 223 - 234, XP005408796
- MASANORI SEIMIYA: 'Genpatsusei Kansaibo Gan Soshiki no Proteome Kaiseki to Byori Soshiki Shindan eno Oyo' RINSHO KAGAKU vol. 38, no. SUPP.L, 2009, pages 5 - 6, XP008164125
- SEIMIYA: 'Identification of Novel Immunohistochemical Tumor Markers for Primary Hepatocellular Carcinoma; Clathrin Heavy Chain and Formiminotransferase Cyclodeaminase' HEPATOLOGY vol. 48, 2008, pages 519 - 530, XP008161291
- New England Biolabs Inc: "Home › Tools & Resources › Affinity of Protein A/G for IgG Types from Different Species Affinity of Protein A/G for IgG Types from Different Species Species Immunoglobulin Binding to Protein A Binding to Protein G" In: "Affinity of Protein A/G for IgG Types from Different Species Affinity of Protein A/G for IgG Types from Different Species Species Immunoglobulin Binding to Protein A Binding to Protein G", 1 January 2016 (2016-01-01), New England BioLabs Inc, Ipswich MA USA, XP055296360, pages 1-1,
- T. SUZUKI ET AL.: "Ligand binding characteristics", J VET MED SCI, vol. 77, no. 11, 2015, Tokyo
- J.A. SCHROER ET AL.: "Mapping epitopes", EUR J IMMUNOL (WILEY-VCH INC), vol. 13, 1983, pages 693-700, Hoboken NJ USA
- Abnova: "Datasheet XRCC5 monoclonal antibody", 8 November 2016 (2016-11-08), Abnova, Taipei

## Description

### TECHNICAL FIELD

The present invention relates to a method of immunoassay for measuring a complex between Ku86 and its autoantibody and a kit for the immunoassay. In particular, a complex between Ku86 and its autoantibody occurs specifically in the blood of carcinoma bearing patients at high levels. Thus, the present invention provides a method of measuring the complex between Ku86 and its autoantibody for determination of carcinoma.

### BACKGROUND ART

Ku86 is a protein which is involved in cleavage of double-stranded DNA, and, together with Ku70, forms a heterodimer, referred to as Ku. That Ku heterodimer has been described as being capable of repairing cleavage of double-stranded DNA in cooperation with a DNA dependent protein kinase and the like (Non Patent Literature 1).

Meanwhile, a modified agarose two-dimensional electrophoresis in which 2D-DIGE technique (two-dimensional fluorescence difference gel electrophoresis) is applied to an agarose two-dimensional electrophoresis (Non Patent Literature 2) has revealed that, by comparing the protein expression levels between a cancerous tissue of primary hepatocellular carcinoma and a peripheral non-cancerous tissue using a proteomic analysis, a protein Ku86 is expressed abundantly in the cancerous area (Non Patent Literature 3).

The scientific publication by Yaneva and Arnett discloses that antibodies against Ku protein are found in sera from patients with autoimmune disease (YANEVA ET AL.: 'Antibodies against Ku protein in sera from patients with autoimmune diseases' CLIN.EXP.IMMUNOL. vol. 76, 1989, pages 366 - 372).

Gullo et al. published a review article relating to the biology of Ku and its potential oncogenic role in cancer (GULLO ET AL.: 'The biology of Ku and its potential oncogenic role in cancer' BIOCHIMICA ET BIOPHYSICA ACTA vol. 1765, 2006, pages 223 - 234).

The publication MASANORI SEIMIYA: 'Genpatsusei Kansaibo Gan Soshiki no Proteome Kaiseki to Byori Soshiki Shindan eno Oyo' RINSHO KAGAKU vol. 38, no. SUPP.L, 2009, pages 5 - 6 is a short communication of Non Patent Literature 3.

WO 2004/048523 A2 relates to compounds, compositions and methods for modulating the expression of Ku86.

WO 2007/115829 A2 relates to an agent comprising a binding moiety capable of selectively binding to Ku protein for use in medicine, in particular in the treatment and diagnosis of cancer.

WO 2011/090017 A1 relates to the measurement of an autoantibody against Ku86 by measuring the immune complex that is formed between the autoantibody contained in a sample and a Ku86 antigen. It is further disclosed that the measurement of said autoantibody enables the determination of primary hepatocellular carcinoma.

Nomura et al. disclose serum anti-Ku86 as a potential biomarker for early detection of hepatitis C virus-related hepatocellular carcinoma (F. NOMURA ET AL: "Serum anti-Ku86 is a potential biomarker for early detection of hepatitis C virus-related hepatocellular carcinoma", BIOCHEMICAL AND BIOPHYSICAL RESEARCH COMMUNICATIONS, vol. 421, no. 4, 25 April 2012 (2012-04-25), pages 837-843).

### CITATION LIST

### Non Patent Literature

Non Patent Literature 1: Li et al., Proc. Natl. Acad. Sci. USA, Vol.9, No.2, 832-837,2002
Non Patent Literature 2: Takeshi Tomonaga et al., Clin. Cancer Res.
Non Patent Literature 3: Masanori Seimiya et al., Hepatology 2008;48:519-30

### SUMMARY OF INVENTION

### TECHNICAL PROBLEM

The present inventors have surprisingly found that a complex between Ku86 and its autoantibody may be present in a blood specimen, and the amount of the complex is abundant in the case of patients with carcinoma. Therefore, an object of the present invention is to provide a method of measuring a complex between Ku86 and its autoantibody, which can be applied to the determination of carcinoma.

### SOLUTION TO PROBLEM

The present inventors have found that a complex between Ku86 and its autoantibody in a specimen can be measured by immunoassaying the complex, thereby allowing determination of carcinoma, and then completed the present invention.

Thus, the present invention relates to a method of immunoassay for a complex between Ku86 and its autoantibody in a specimen, comprising immunoassaying the complex between Ku86 and its autoantibody in the specimen for determination of carcinoma.

Furthermore, the present invention relates to a kit for immunoassay of a complex between Ku86 and its autoantibody, comprising a reagent antibody against Ku86 and a substance capable of binding to the autoantibody.

Furthermore, the present invention relates to a method of determining carcinoma, wherein the presence of the carcinoma is determined by measuring a complex between Ku86 and its autoantibody.

Furthermore, the present invention relates to a marker for determining carcinoma, comprising a complex between Ku86 and its autoantibody.

### ADVANTAGEOUS EFFECTS OF INVENTION

In the present invention, a complex between Ku86 and its autoantibody present in a specimen derived from blood ma be easil measured, which is effective for determination of a patient with carcinoma selected from the group consisting of primary hepatocellular carcinoma, colorectal carcinoma, gastric carcinoma, pancreatic carcinoma, breast carcinoma, lung carcinoma, and esophageal carcinoma.

### BRIEF DESCRIPTION OF DRAWINGS

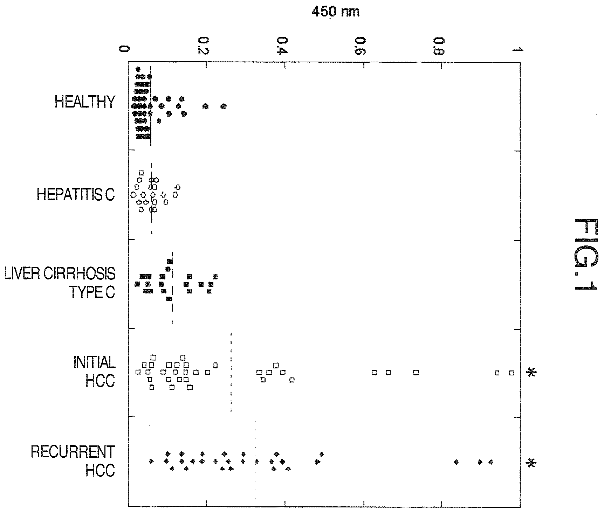

[Fig. 1] Fig. 1 shows results of measurement of a complex between Ku86 and its autoantibody in serum specimens from healthy individuals, serum specimens from patients with hepatitis C, serum specimens from patients with liver cirrhosis type C, serum specimens from patients with initial primary hepatocellular carcinoma (hereinafter, it may be referred to as initial HCC) and serum specimens from patients with recurrent primary hepatocellular carcinoma (hereinafter, it may be referred to as recurrent HCC), using an ELISA plate sensitized with an anti-Ku86 antibody. In the figure, the horizontal axis represents disease names, and the vertical axis represents absorbance for light with wavelength of 450 nm. An asterisk represents the group of serum specimen in which there are significant difference (p<0.0001) according to Wilcoxon two sample test in all cases when compared to the group of serum specimens from healthy individuals, the group of specimens from patients with hepatitis C or the group of serum specimens from patients with liver cirrhosis type C.

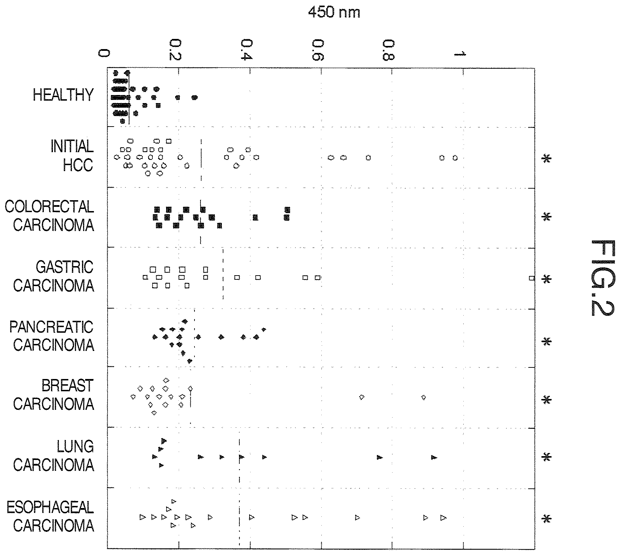

[Fig. 2] Fig. 2 shows results of measurement of an complex between Ku86 and its autoantibody in serum specimens from healthy individuals, serum specimens from patients with initial HCC, serum specimens from patients with colorectal carcinoma, serum specimens from patients with gastric carcinoma, serum specimens from patients with pancreatic carcinoma, serum specimens from patients with breast carcinoma, serum specimens from patients with lung carcinoma, and serum specimens from patients with esopltageal carcinoma, using an ELISA plate sensitized with an anti-Ku86 antibody. In the figure, the horizontal axis represents disease names, and the vertical axis represents absorbance for light with wavelength of 450 nm. An asterisk represents the group of serum specimens in which there are significant difference (p<0.0001) according to Wilcoxon two sample test in all cases when compared to the group of serum specimens from healthy individuals.

### DESCRIPTION OF EMBODIMENTS

Known methods of immunoassay for immunocomplexes between common proteins and their antibodies can be directly applied to the inventive method of immunoassay for a complex between Ku86 and its autoantibody.

In the method of immunoassay of the present invention, a complex between Ku86 and its autoantibody is measured, for example, by reacting the complex in a specimen with a reagent antibody against Ku86 and a substance capable of binding to the autoantibody, and measuring the resulting immunocomplex among the complex, the reagent antibody and the bindable substance.

In the present invention, a specimen is a specimen derived from blood and examples of a blood specimen include whole blood, blood plasma, and serum.

The subject to be measured by the method of immunoassay of the present invention is a complex between Ku86 and its autoantibody. As described above, Ku86 is a protein which is involved in cleavage of double-stranded DNA, its formal name being ATP-dependent DNA helicase 2 subunit 2. It is otherwise referred to as XRCC5. Additionally, Ku86 is an 82 kDa protein consisting of 732 amino acids, and its accession number (accession No) in US National Center for Biotechnology Information (NCBI) is gi-10863945.

In the present invention, a complex between Ku86 and its autoantibody means an immunocomplex between Ku86 and an autoantibody against Ku86, and may be described herein simply as the complex.

In the present invention, an autoantibody refers to an antibody that is produced by one's own body against substances present in its own body, wherein the substance present in one's own body corresponds to Ku86, and the autoantibody corresponds to an antibody against Ku86.

In the present invention, a reagent antibody against Ku86 refers to an antibody used as a reagent that specifically binds to Ku86, and may be described herein simply as a reagent antibody. That reagent antibody is produced by animal species such as human, mouse, rat, rabbit, goat, and horse, each producing a given class of immunoglobulin. The reagent antibody may be any of IgG, IgM, IgA, IgE, and IgD. Also, the reagent antibody may be any of a monoclonal antibody, a polyclonal antibody, and fragments thereof (those having an ability to bind to an antigen, for example, H-chain, L-chain, Fab, and F(ab')2). These reagent antibodies can be obtained as antiserums from the immunized animals generated by immunizing the above described animal species producing antibodies with Ku86 full length proteins or fragment peptides thereof as antigens, or as monoclonal antibodies from the hybridomas obtained by fusing splenocytes from immunized animals and myeloma cells, and screening the fused cells for those producing an antibody against Ku86. As the reagent antibody against Ku86, those products commercially available as an anti-Ku86 antibody may also be used.

In the present invention, a substance capable of binding to the autoantibod is an anti-IgG antibody, Protein A, Protein G, or a Ku86 antigen and among them an anti-IgG antibody is preferred.

When using a Ku86 antigen as a reagent, though it is not especially limited as long as it is an antigen having antigenic reactivity to an autoantibody against Ku86, examples of it may include a Ku86 full length protein, a variant of the Ku86 full length protein which is a protein retaining the equivalent antigenic reactivity of the full length protein to an autoantibody against Ku86, and having a homology of 90% or more to the amino acid sequence, or a variant protein having an amino acid sequence resulting from deletion, substitution, or addition of one to several amino acid residues in the amino acid sequence of the Ku86 full length protein, and a fragment peptide of Ku86 having antigenic reactivity to an autoantibody against Ku86. Though a Ku86 full length protein is available from Abnova Corporation, the protein or its variant may be synthesized by genetic engineering techniques as its entire amino acid sequence is known. When used in the present invention, a fragment peptide of Ku86 may be generated by cleaving a Ku86 full length protein into various peptide fragments by enzymatic hydrolysis and the like, or may also be easily generated using a commercial automated peptide synthesizer. Additionally, the targeted fragment peptide of Ku86 may be generated by genetic engineering techniques.

The thus-obtained variant or fragment peptide of the Ku86 full length protein may be reacted with an autoantibody against Ku86, then those having antigenic reactivity may be selected to be used as a Ku86 antigen as a reagent. In the present invention, the whole of the each peptide fragment above described, as well as a part of it and the mixture thereof may be used, and are included in a Ku86 antigen as a reagent.

In the present invention, reacting a complex between Ku86 and its autoantibody in a specimen with a reagent antibody against Ku86 and a substance capable of binding to the autoantibody generates an immunocomplex among the complex, the reagent antibody and the bindable substance. To measure the immunocomplex, the immunocomplex may be preferably measured by labeling either of the reagent antibody against Ku86 (the reagent antibody) or the substance capable of binding to the autoantibody (the bindable substance) with a labeling component, and measuring the labeling component in the generated immunocomplex.

As a labeling component, labeling components that are routinely used, such as enzymes, radioactive substances, fluorescent substances, and chemiluminescent substances may be used, but enzymes and radioactive substances are preferred.

As an enzyme used for labeling, an enzyme that is routinely used in enzyme immunoassay (EIA), such as horseradish peroxidase, calf intestine alkaline phosphatase, β-galactosidase, urease, or glucose oxidase may be used as appropriate, and chromogenic substrates adapted to these enzymes and used routinely in EIA may be used as appropriate. As chromogenic substrates, for example, in the case of HRP, 3, 3', 5, 5'-tetramethyl benzidine (TMBZ), TMBZ·HCl, TMBZ·PS, ABTS, o-phenylene diamine, p-hydroxyphenyl acetic acid and the like may be used, in the case of alkaline phosphatase, p-nitrophenyl phosphate, 4-methylumbelliferyl phosphate and the like may be used, and in the case of (β-galactosidase, o-nitrophenyl-β-D-galactopyranoside, 4-methylumbelliferyl (β-D-galactopyranoside and the like may be used.

As examples of radioactive substances used for labeling, for example, a radioactive iodine atom, as fluorescent substances, for example, FITC and rhodamine, and as chemiluminescent substances, for example, luminol may be included.

When using a labeling component in the present invention, for example, the complex is preferably measured by reacting a complex between Ku86 and its autoantibody in a specimen with a reagent antibody against Ku86 bound to a water-insoluble carrier, subsequently reacting the resulting product with a substance capable of binding to the autoantibody, labeled with the labeling component, to generate an immunocomplex among the complex, the reagent antibody, and the bindable substance, and measuring the labeling component bound to the immunocomplex. Alternatively, the complex may be measured by reacting a complex between Ku86 and its autoantibody in a specimen with a substance capable of binding to the autoantibody bound to a water-insoluble carrier, subsequently reacting the resulting product with a reagent antibody against Ku86 labeled with the labeling component, to generate an immunocomplex among the complex, the bindable substance, and the reagent antibody, and measuring the labeling component bound to the immunocomplex.

The preparation of a water-insolubilized carrier may be easily carried out using a known method that binds a protein to the surface of a solid phase. As a solid-phased carrier, for example, beads, microplates, tubes and the like are generally used. For a method of binding a reagent antibody against Ku86 to the surface of these solid-phased carriers, known immobilization techniques such as physisorption and chemical binding may be utilized as appropriate.

Upon contacting the thus solid-phased reagent antibody against Ku86 with a specimen comprising a complex between Ku86 and its autoantibody, the Ku86 portion of then complex binds to the reagent antibody. Further, reacting the bound product with a substance capable of binding to the autoantibody and labeled with a labeling substance (for example, a labeled anti-IgG antibody) generates the immunocomplex among the complex, the reagent antibody and the bindable substance. Consequently, the complex between Ku86 and its autoantibody in the specimen can be measured by measuring the labeling component in the generated immunocomplex.

In a similar way to the above, by binding a substance capable of binding to Ku86 autoantibody to a solid-phased carrier, contacting the solid-phased bindable substance with a specimen comprising a complex between Ku86 and its autoantibody, to allow binding of the autoantibody portion of the complex to the bindable substance, and further reacting the bound product with the reagent antibody against Ku86 and labeled with a labeling substance (for example, an anti-Ku86 antibody) to generate the immunocomplex among the complex, the bindable substance and the reagent antibody, the complex between Ku86 and its autoantibody in the specimen may likewise be measured.

Typical examples of the method of immunoassay of the present invention are shown below.

An anti-Ku86 antibody is added to a plate, the plate is left at rest at low temperature, for example, 4°C for sensitization, and subsequently washed with a washing solution such as PBS. That plate is then coated with BSA to generate an anti-Ku86 antibody ELISA plate. A diluted specimen is added to the anti-Ku86 antibody ELISA plate, the plate is left at rest at warm temperature, for example at 37°C, and then washed with a washing solution such as PBS. An HRP-labeled anti-Human IgG antibody is added to wells in the resulting plate, the plate is left at rest at warm temperature, for example at 37°C. Then, after washing the wells with a washing solution such as PBS, TMBZ is added thereto, left at rest for example at room temperature, and subsequently 1 N sulfuric acid is added as quencher. For absorbance, absorbance at the wavelength of 450 nm is measured using a microplate reader (from BioRad Laboratories Inc.). The value of a complex between Ku86 and its autoantibody is determined from the absorbance values and the previously made calibration curve.

The method of measuring of the present invention may be performed by a kit for immunoassay of a complex between Ku86 and its autoantibody, comprising a reagent antibody against Ku86 and a substance capable of binding to the autoantibody. The reagent antibody against Ku86 and the substance capable of binding to the autoantibody for this purpose are as described in the method of measuring of the present invention. That is to say, for example, the reagent antibody against Ku86 and the substance capable of binding to the autoantibody, either of which is in a form bound to a water-insoluble carrier, and the other one in a form labeled with a labeling component, may be reagent components of the kit. As additional reagent components, those used routinely in immunoassay such as surfactants, and buffers may be added as appropriate.

In the present invention, carcinoma can be determined by measuring a complex between Ku86 and its autoantibody. Utilizing the method of measuring of the present invention is effective for distinguishing carcinomas selected from the group consisting primary hepatocellular carcinoma, colorectal carcinoma, gastric carcinoma, pancreatic carcinoma, breast carcinoma, lung carcinoma and esophageal carcinoma. For example, it is effective for distinguishing healthy individuals from patients with initial primary hepatocellular carcinoma, patients with recurrent primary hepatocellular carcinoma, patients with colorectal carcinoma, patients with gastric carcinoma, patients with pancreatic carcinoma , patients with breast carcinoma, patients with lung carcinoma or patients with esophageal carcinoma. Also, by utilizing the method of measuring of the present invention, it is possible to distinguish a patient with primary hepatocellular carcinoma such as a patient with new-onset primary hepatocellular carcinoma or a patient with recurrent primary hepatocellular carcinoma from a patient with hepatitis C or a patient with liver disease such as liver cirrhosis type C.

As apparent from the description above, a complex between Ku86 and its autoantibody may be used as a marker for determination of carcinoma using a specimen derived from blood such as whole blood, blood plasma, serum wherein the carcinoma is selected from the group of carcinomas as defined in claim 9.

### EXAMPLES

Hereinafter, the present invention will be described in more detail with reference to the examples, but the present invention is not to be limited to these examples in any way.

### Example 1

### Measurement of a complex between Ku86 and its autoantibody,

For serum specimens collected from healthy individuals, patients with hepatitis C, patients with liver cirrhosis type C, patients with initial primary hepatocellular carcinoma, patients with recurrent primary hepatocellular carcinoma, patients with colorectal carcinoma, patients with gastric carcinoma, patients with pancreatic carcinoma, patients with breast carcinoma, patients with lung carcinoma, and patients with esophageal carcinoma, a complex between Ku86 and its autoantibody was measured in the manner described specifically below.

### 1. Method

### (1) Generation of an anti-Ku86 antibody ELISA plate

An ELISA plate (from Nunc International, Maxisorp) was sensitized by leaving at rest an anti-XRCC5 antibody(from Abnova Corporation, 5 µg/ml, 100 µL/well)as an anti-Ku86 antibody on the plate overnight at 4°C, and then washed with PBS containing 0.05% Tween20 (200µL/well) three times. The plate was then coated overnight with PBS containing 1.5% BSA, 10% saccharose (200µL/well) to generate the anti-Ku86 antibody ELISA plate.

### (2) Measurement of the complex between Ku86 and its autoantibody

An HRP-labeled anti-Human IgG antibody (from Zymed Laboratories Inc.) as a detection antibody diluted 4000 times with PBS containing 0.05% Tween20 was used. Sample serum was diluted 100 times with PBS. The diluted sample was added to the anti-Ku86 antibody ELISA plate in an amount of 100 µL/well, the plate was left at rest for 1 hour at 37°C, and then washed with PBS containing 0.05% Tween20 (200 µL/well) 3 times. To each well of the resulting plate, 100 µL/well of a diluted HRP-labeled anti-Human IgG antibody was added, the plate was left at rest for 30 min at 37°C. Then, after washing the plate with PBS containing 0.05% Tween20 (200 µL/well) three times, TMBZ was added to the plate in an amount of 100 µL/well. After leaving the plate at rest for 10 minutes at room temperature, 100 µL/well of 1 N sulfuric acid as a quencher was added to the plate. Absorbance was measured using a microplate reader (from BioRad Laboratories Inc.) at the wavelength of 450 nm.

It is noted that specimens from 48 healthy individuals, 19 hepatitis C patients, 18 liver cirrhosis type C patients, 32 specimens from new-onset primary hepatocellular carcinoma patients, 27 recurrent primary hepatocellular carcinoma patients, 16 specimens from colorectal carcinoma patients, 16 gastric carcinoma patients, 16 pancreatic carcinoma patients, 16 breast carcinoma patients, 16 lung carcinoma patients, 16 esophageal carcinoma patients were used.

### 2. Results

Results of the measurement of a complex between Ku86 and its autoantibody were shown in Fig. 1 and Fig. 2. For significant difference test, KaleidaGraph 4.0 was used, and statistical processing was carried out with Wilcoxon two sample test.

As shown in Fig. 1, as compared to the group of healthy individuals, the group of hepatitis C and the group of liver cirrhosis type C, obvious significant differences in the amounts of the complex between Ku86 and its autoantibody were observed in the group of specimen from patients with initial primary hepatocellular carcinoma and the group of specimen from patients with recurrent primary hepatocellular carcinoma. Therefore, immunoassay of the complex between Ku86 and its autoantibody in blood was shown to be particularly effective for distinguishing primary hepatocellular carcinoma among liver diseases.

As shown in Fig. 2, as compared to the group of healthy individual, obvious significant differences in the amounts of the complex between Ku86 and its autoantibody were observed in the group of specimens from patients with various carcinoma, i.e., the group of specimens from patients with initial primary hepatocellular carcinoma, the group of specimens from patients with colorectal carcinoma, the group of specimens from patients with gastric carcinoma, the group of specimens from patients with pancreatic carcinoma, the group of specimens from patients with breast carcinoma, the group of specimens from patients with lung carcinoma and the group of specimens from patients with esophageal carcinoma. Therefore, immunoassay of the complex between Ku86 and its autoantibody in blood was shown to be effective for distinguishing a patient with carcinoma from healthy individuals, in various carcinomas.

### INDUSTRIAL APPLICABILITY

As described in detail hereinbefore, a complex between Ku86 and its autoantibody can be measured by reacting the complex in a specimen such as a specimen derived from blood with a reagent antibody against Ku86 and a substance capable of binding to the autoantibody, and measuring the resulting immunocomplex among the complex, the reagent antibody and the bindable substance, thereby allowing determination of carcinoma selected from the group consisting of primary hepatocellular carcinoma, colorectal carcinoma, gastric carcinoma, pancreatic carcinoma, breast carcinoma, lung carcinoma and esophageal carcinoma.

## Claims

1. An in vitro method of measuring a complex between human Ku86 and its autoantibody present in a blood specimen comprising
(i) reacting the Ku86-autoantibody complex in said specimen with a reagent antibody against Ku86 and a substance capable of binding to the autoantibody, thereby generating an immunocomplex comprising said Ku86-autoantibody complex, the reagent antibody and the substance capable of binding to the autoantibody,
wherein the substance capable of binding to the autoantibody is anti- IgG antibody and
wherein
(a) the reagent antibody is bound to a water-insoluble carrier, and the substance capable of binding to the autoantibody is labeled with a labeling component or
(b) the reagent antibody is labeled with a labeling component, and the substance capable of binding to the autoantibody is bound to a water-insoluble carrier, and
(ii) measuring the Ku86-autoantibody complex by measuring the labeling component bound to the immunocomplex generated in step (i),
and wherein the reagent antibody against Ku86 is produced by mouse, rat, rabbit, goat or horse.

2. The method according to claim 1, wherein the Ku86-autoantibody complex is measured by reacting the Ku86-autoantibody complex present in the specimen with the reagent antibody against Ku86 bound to the water-insoluble carrier, subsequently reacting the carrier-bound product with the substance capable of binding to the autoantibody and labeled with the labeling component,
to generate the immunocomplex among the Ku86-autoantibody complex, the reagent antibody, and the substance capable of binding to the autoantibody, and measuring the labeling component in the carrier-bound immunocomplex generated in step (i).

3. The method according to claim 1, wherein the Ku86-autoantibody complex is measured by
reacting the Ku86-autoantibody complex present in the specimen with the substance capable of binding to the autoantibody and bound to the water-insoluble carrier,
subsequently reacting the carrier-bound product with the reagent antibody against Ku86 labeled with the labeling component,
to generate the immunocomplex among the Ku86-autoantibody complex, the reagent antibody, and the substance capable of binding to the autoantibody, and measuring the labeling component in carrier-bound immunocomplex generated in step (i)

4. The method according to any one of claims 1 to 3, wherein the labeling component is an enzyme or a radioactive substance.

5. A method for determination of carcinoma comprising the method according to any one of claims 1 to 4, wherein the carcinoma is selected from the group consisting of primary hepatocellular carcinoma, colorectal carcinoma, gastric carcinoma, pancreatic carcinoma, breast carcinoma, lung carcinoma and esophageal carcinoma,
wherein the abundance of the complex between Ku86 and its autoantibody is used as a marker for determining carcinoma.

6. Use of a kit in the method of any one of claims 1 to 5,
wherein the kit comprises a reagent antibody against human Ku86; and an anti-IgG antibody as the substance capable of binding to Ku86 autoantibody,
wherein the reagent antibody is antibody against Ku86 is produced by mouse, rat, rabbit, goat or horse.

7. In vitro use of a complex between Ku86 and its autoantibody as a marker for determining carcinoma, wherein the specimen for determining the carcinoma is derived from blood; and
wherein the carcinoma is selected from the group consisting of primary hepatocellular carcinoma, colorectal carcinoma, gastric carcinoma, pancreatic carcinoma, breast carcinoma, lung carcinoma and esophageal carcinoma.

8. Marker for use in a method for determining carcinoma;
wherein the marker comprises a complex between Ku86 and its autoantibody;
wherein the carcinoma is selected from the group consisting of primary hepatocellular carcinoma, colorectal carcinoma, gastric carcinoma, pancreatic carcinoma, breast carcinoma, lung carcinoma and esophageal carcinoma; and
wherein the method is a method practised on the human or animal body.

## Patentansprüche

1. *In vitro* Verfahren zum Messen eines in einer Blutprobe vorliegenden Komplexes zwischen menschlichem Ku86 und seinem Autoantikörper, umfassend
(i) Reagieren des Ku86-Autoantikörper-Komplexes in der Probe mit einem Reagenzantikörper gegen Ku86 und einer Substanz, die in der Lage ist an den Autoantikörper zu binden, wodurch ein Immunkomplex, umfassend den Ku86-Autoantikörper-Komplex, den Reagenzantikörper und die Substanz, die in der Lage ist an den Autoantikörper zu binden, gebildet wird,
wobei die Substanz, die in der Lage ist an den Autoantikörper zu binden, anti-IgG-Antikörper ist und wobei
(a) der Reagenzantikörper an einen wasserunlöslichen Träger gebunden ist und die Substanz, die in der Lage ist an den Autoantikörper zu binden, mit einer Markierungskomponente markiert ist, oder
(b) der Reagenzantikörper mit einer Markierungskomponente markiert ist und die Substanz, die in der Lage ist an den Autoantikörper zu binden, an einen wasserunlöslichen Träger gebunden ist, und
(ii) Messen des Ku86-Autoantikörper-Komplexes durch das Messen der Markierungskomponente, die an den in Schritt (i) gebildeten Immunkomplex gebunden ist,
und wobei der Reagenzantikörper gegen Ku86 durch Maus, Ratte, Kaninchen, Ziege oder Pferd produziert ist.

2. Verfahren gemäß Anspruch 1, wobei der Ku86-Autoantikörper-Komplex gemessen wird,
indem der in der Probe vorliegende Ku86-Autoantikörper-Komplex mit dem an den wasserunlöslichen Träger gebundenen Reagenzantikörper gegen Ku86 reagiert wird, danach das Träger-gebundene Produkt mit der Substanz, die in der Lage ist an den Autoantikörper zu binden und die mit der Markierungskomponente markiert ist, reagiert wird,
um den Immunkomplex zwischen dem Ku86-Autoantikörper-Komplex, dem Reagenzantikörper und der Substanz, die in der Lage ist an den Autoantikörper zu binden, zu bilden, und die Markierungskomponente im in Schritt (i) gebildeten Träger-gebundenen Immunkomplex gemessen wird.

3. Verfahren gemäß Anspruch 1, wobei der Ku86-Autoantikörper-Komplex gemessen wird,
indem der in der Probe vorliegende Ku86-Autoantikörper-Komplex mit der Substanz, die in der Lage ist an den Autoantikörper zu binden und die an den wasserunlöslichen Träger gebunden ist, reagiert wird, danach das Träger-gebundene Produkt mit dem mit der Markierungskomponente markierten Reagenzantikörper gegen Ku86 reagiert wird,
um den Immunkomplex zwischen dem Ku86-Autoantikörper-Komplex, dem Reagenzantikörper und der Substanz, die in der Lage ist an den Autoantikörper zu binden, zu bilden, und die Markierungskomponente im in Schritt (i) gebildeten Träger-gebundenen Immunkomplex gemessen wird.

4. Verfahren gemäß irgendeinem der Ansprüche 1 bis 3, wobei die Markierungskomponente ein Enzym oder eine radioaktive Substanz ist.

5. Verfahren zum Bestimmen von Karzinom, umfassend das Verfahren gemäß irgendeinem der Ansprüche 1 bis 4, wobei das Karzinom ausgewählt ist aus der Gruppe, bestehend aus primärem hepatozellulärem Karzinom, kolorektalem Karzinom, Magenkarzinom, Pankreaskarzinom, Brustkarzinom, Lungenkarzinom und Ösophaguskarzinom, wobei die Menge des Komplexes zwischen Ku86 und seinem Autoantikörper als ein Marker zum Bestimmen von Karzinom verwendet wird.

6. Verwendung eines Kits im Verfahren gemäß irgendeinem der Ansprüche 1 bis 5,
wobei das Kit einen Reagenzantikörper gegen menschliches Ku86; und einen anti-IgG-Antikörper als die Substanz, die in der Lage ist an den Ku86-Autoantikörper zu binden, umfasst,
wobei der Reagenzantikörper ein Antikörper gegen Ku86 ist, der durch Maus, Ratte, Kaninchen, Ziege oder Pferd produziert ist.

7. *In vitro* Verwendung eines Komplexes zwischen Ku86 und seinem Autoantikörper als ein Marker zum Bestimmen von Karzinom, wobei die Probe zum Bestimmen des Karzinoms aus Blut abgeleitet ist; und
wobei das Karzinom ausgewählt ist aus der Gruppe, bestehend aus primärem hepatozellulärem Karzinom, kolorektalem Karzinom, Magenkarzinom, Pankreaskarzinom, Brustkarzinom, Lungenkarzinom und Ösophaguskarzinom.

8. Marker zur Verwendung in einem Verfahren zum Bestimmen von Karzinom;
wobei der Marker einen Komplex zwischen Ku86 und seinem Autoantikörper umfasst;
wobei das Karzinom ausgewählt ist aus der Gruppe, bestehend aus primärem hepatozellulärem Karzinom, kolorektalem Karzinom, Magenkarzinom, Pankreaskarzinom, Brustkarzinom, Lungenkarzinom und Ösophaguskarzinom; und wobei das Verfahren ein Verfahren ist, das am menschlichen oder tierischen Körper vorgenommen wird.

## Revendications

1. Procédé *in vitro* de mesure d'un complexe entre la Ku86 humaine et son auto-anticorps présent dans un échantillon de sang, comprenant
(i) la mise en réaction du complexe Ku86-auto-anticorps dans ledit échantillon avec un anticorps réactif dirigé contre Ku86 et une substance capable de se lier à l'auto-anticorps, en générant ainsi un complexe immun comprenant ledit complexe Ku86-auto-anticorps, l'anticorps réactif et la substance étant capable de se lier à l'auto-anticorps,
où la substance capable de se lier à l'auto-anticorps est un anticorps anti-IgG et dans lequel
(a) l'anticorps réactif est lié à un support insoluble dans l'eau, et la substance capable de se lier à l'auto-anticorps est marquée avec un composant de marquage ou
(b) l'anticorps réactif est marqué avec un composant de marquage, et la substance capable de se lier à l'auto-anticorps est liée à un support insoluble dans l'eau,
et
(ii) la mesure du complexe Ku86-auto-anticorps en mesurant le composant de marquage lié au complexe immun généré à l'étape (i),
et dans lequel l'anticorps réactif dirigé contre Ku86 est produit par une souris, un rat, un lapin, une chèvre ou un cheval.

2. Procédé selon la revendication 1, dans lequel le complexe Ku86-auto-anticorps est mesuré par la mise en réaction du complexe Ku86-auto-anticorps présent dans l'échantillon avec l'anticorps réactif dirigé contre Ku86 lié au support insoluble dans l'eau, puis par la mise en réaction du produit lié au support avec la substance capable de se lier à l'auto-anticorps et marquée avec le composant de marquage,
afin de générer le complexe immun parmi le complexe Ku86-auto-anticorps, l'anticorps réactif, et la substance capable de se lier à l'auto-anticorps, et la mesure du composant de marquage dans le complexe immun lié au support généré à l'étape (i).

3. Procédé selon la revendication 1, dans lequel le complexe Ku86-auto-anticorps est mesuré par
la mise en réaction du complexe Ku86-auto-anticorps présent dans l'échantillon avec la substance capable de se lier à l'auto-anticorps et liée au support insoluble dans l'eau,
puis la mise en réaction du produit lié au support avec l'anticorps réactif dirigé contre Ku86 marqué avec le composant de marquage,
afin de générer le complexe immun parmi le complexe Ku86-auto-anticorps, l'anticorps réactif, et la substance capable de se lier à l'auto-anticorps, et la mesure du composant de marquage dans le complexe immun lié au support généré à l'étape (i).

4. Procédé selon l'une quelconque des revendications 1 à 3, dans lequel le composant de marquage est une enzyme ou une substance radioactive.

5. Procédé pour la détermination d'un carcinome comprenant le procédé selon l'une quelconque des revendications 1 à 4, dans lequel le carcinome est sélectionné dans le groupe consistant en un carcinome hépatocellulaire primitif, un carcinome colorectal, un carcinome gastrique, un carcinome pancréatique, un carcinome mammaire, un carcinome pulmonaire et un carcinome oesophagien,
dans lequel l'abondance du complexe entre Ku86 et son auto-anticorps est utilisée en tant que marqueur pour déterminer le carcinome.

6. Utilisation d'un nécessaire dans le procédé selon l'une quelconque des revendications 1 à 5,
dans laquelle le nécessaire comprend un anticorps réactif dirigé contre la Ku86 humaine ; et un anticorps anti-IgG en tant que substance capable de se lier à l'auto-anticorps de Ku86,
dans laquelle l'anticorps réactif est un anticorps dirigé contre Ku86 qui est produit par une souris, un rat, un lapin, une chèvre ou un cheval.

7. Utilisation *in vitro* d'un complexe entre Ku86 et son auto-anticorps en tant que marqueur pour déterminer un carcinome, dans laquelle l'échantillon pour déterminer le carcinome est dérivé du sang ; et
dans laquelle le carcinome est sélectionné dans le groupe consistant en un carcinome hépatocellulaire primitif, un carcinome colorectal, un carcinome gastrique, un carcinome pancréatique, un carcinome mammaire, un carcinome pulmonaire et un carcinome oesophagien.

8. Marqueur destiné à être utilisé dans un procédé pour déterminer un carcinome;
où le marqueur comprend un complexe entre Ku86 et son auto-anticorps ;
où le carcinome est sélectionné dans le groupe consistant en un carcinome hépatocellulaire primitif, un carcinome colorectal, un carcinome gastrique, un carcinome pancréatique, un carcinome mammaire, un carcinome pulmonaire et un carcinome oesophagien; et
où le procédé est un procédé mis en oeuvre sur le corps humain ou animal.
